# EUROPEAN PATENT APPLICATION

(11) **EP 0 521 421 A2**
(43) Date of publication of application: **07.01.1993**
(21) Application number: 92110920.3
(22) Date of filing: 27.06.1992
(51) Int. Cl.: G01N 33/543

(54) **Method for analysis of liquid samples and substrate for analysis of liquid sample employed in the method**

(30) Priority: 27.06.1991 JP 183256/91
(71) Applicant: IDEMITSU PETROCHEMICAL COMPANY LIMITED, Tokyo 100 (JP)
(72) Inventor: Takase, Minoru, c/o Idemitsu Petrochem. Co. Ltd., Sodegaura-shi, Chiba-ken (JP); Shibata, Kazunori, c/o Idemitsu Petrochem. Co. Ltd, Sodegaura-shi, Chiba-ken (JP)
(74) Representative: Hoeger, Stellrecht & Partner

(57) **Abstract**

A reagent for analysis (e.g. an antibody) is fixed on a rotatable disk 100 having a plurality of channels 102. The flow paths of the channels, inclusive of reagent fixing portions 103, are treated with an aqueous solution consisting essentially of protein so that the contact angle with water is not more than 70° (104). The sample for analysis, that is a liquid sample containing an antigen, is dripped on each channel or the disk is rotated for developing the sample for analysis for reacting the reagent for analysis and the liquid sample by way of an antigen-antibody reaction, and changes produced by the reaction are measured on the disk for analysis. This results in improved fluidity of the sample for analysis in the disk-shaped sensor to improve analytic accuracy and reproducibility.

## Description

### BACKGROUND OF THE INVENTION

### Field of the Invention

This invention relates to a method for analysis of liquid samples and a substrate or such analysis. More particularly, it relates to a method for analysis and a substrate for such analysis whereby analysis accuracy and reproducibility may be improved.

### Description of the Related Art

Recently, with a view to early discovery of diseases, quantitative analyses of trace amounts of components in a sample fluid are carried out frequently, in the same manner as analyses of trace amounts of samples in the field of biotechnology. In keeping with strong demands raised for analyses of liquid samples with high accuracy, various proposals have been made recently.

As a result of our eager searches in this line, the present inventors have found that, by previously coating a reagent on a rotatable disk, reacting a liquid sample with the reagent on the disk and measuring the properties of a reaction product, analyses of the liquid sample may be achieved with high accuracy and efficiency. Thus the methods for analyses of liquid samples with a disk have been arrived at and applied for patent by JP Patent Applications Nos.1-52759(1989), 1-92367 (1989) and 2-270900 (1990).

Meanwhile, with the analyses using the disk coated with the reagent (or a disk-shaped sensor), it is necessary for the samples for analyses or detection or the reagents for analyses to be allowed to flow smoothly to be developed thereon without flow-out on a plurality of sample developing surfaces formed on the surface of the disk which is formed of e.g. plastics.

However, with the above described conventional analysis for liquid samples, the surface of the disk formed of plastics, such as polycarbonate (PC) or polystyrene (PS), exhibits high hydrophobicity, so that a larger centrifugal force is required to fluidize the sample for analysis. On the other hand, because of this larger centrifugal force, it is extremely difficult to control the flow of the sample for analyses which has once started to flow, such that analytic accuracy and reproducibility cannot be increased satisfactorily.

In general, plasma discharge processing or chemical modification operations are used for surface modification of plastics. However, these processing operations are not only in need of special apparatus but are unable to maintain the effects of modification for long without considerable difficulties, although the wetting characteristics of the plastics surface can be improved transiently.

### SUMMARY OF THE INVENTION

### Object of the Invention

In view of the above depicted status of the art, it is an object of the present invention to provide a method for analysis of a liquid sample and a substrate for analyses whereby the sample for analyses put on a disk-shaped sensor may be improved in fluidity for improving analytic accuracy and reproducibility.

### Feature of the Invention

For accomplishing the above object, the present invention provides a method for analyzing a liquid sample comprising fixing a reagent for analysis on a rotatable substrate, developing a liquid sample on the substrate by dripping or rotation of the substrate for reacting the reagent for analysis with the liquid sample and measuring changes produced by the reaction on said substrate, said method further comprising treating a substrate surface inclusive of portions having said reagent for analysis fixed thereon with an aqueous solution consisting essentially of protein, preferably albumin or casein, so that the contact angle of the substrate in water is not more than 70° and preferably in the range of from 60° to 50° , and subsequently analyzing said liquid sample.

According to the present invention, the disk-shaped sensor may be improved in fluidity and non-specific reactions may be inhibited to improve analytic accuracy and reproducibility.

### BRIEF DESCRIPTION OF THE DRAWINGS

Fig.1 is a plan view showing an example of a disk-shaped sensor according to the present invention.

Fig.2 is a schematic view showing a typical apparatus for analysis employed in the method of the present invention.

Fig.3 is a schematic view showing the manner of measurement by optical analytic means.

Fig.4 is a graph showing a method for calculating the intensity of the fluorescent light in terms of the numbers of particles.

Fig.5 is a graph showing a calibration curve for CRP found in an Example.

Fig.6 is a graph showing a calibration curve for CRP found in a Comparative Example.

Figs.7(a) and 7(b) are plan views showing the states of development of samples for analysis in the Example and in the Comparative Example.

### DETAILED DESCRIPTION OF THE INVENTION

The present invention will be explained hereinbelow in detail.

First, a substrate for analysis for a liquid sample of the present invention will be explained.

The substrate for analysis of liquid samples according to the present invention is prepared by processing a substrate surface, (on which a reagent (103) for analyses is fixed.) of a rotatable substrate with an aqueous solution of a protein (104).

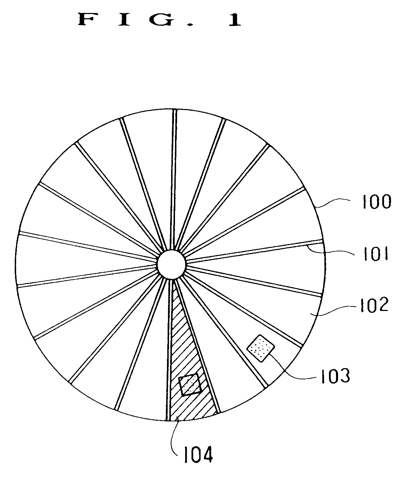

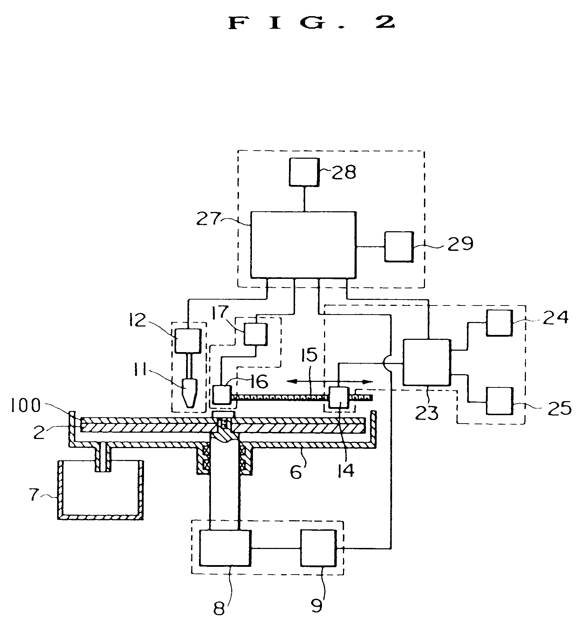

Although there is no limitation as to the size or thickness of the substrate (disk), the substrate needs to be rotatable so as to be suited for sample development and analyses by optical means. For this reason, a disk-shaped substrate is most preferred. As shown in fig.1, the substrate 100 is preferably formed with a large number of radially extending ribs 101 to form a large number of sample-developing surfaces 102 and reaction sites 103 on each of which an antibody is fixed to permit a large number of samples to be analyzed simultaneously. The substrate 100 may be formed of transparent materials, such as plastics, e.g. polycarbonate (PC), polystyrene (PS), polymethyl methacrylate, polyvinyl chloride, polyurethane or epoxy resins, or glass. Of these, polycarbonate and polymethyl methacrylate are most preferred. The substrate is formed of a transparent material in this manner because it permits of optical analyses by a white light source or laser.

A reagent for analysis fixed on the substrate 100 is different according to the item to be measured.

For carrying out an analysis using an antigen-antibody reaction, an antibody or an antigen showing reaction specificity with respect to the sample to be analyzed (antigen or antibody) is used as a reagent for analysis.

Specifically, if the sample to be analyzed is an antigen, a polyclonal antibody or a monoclonal antibody, produced by administering the antigen to an animal, such as rabbit, goat or sheep, is used as a reagent for analyses.

For fixing the antibody to the substrate 100, a 0.05 M tris buffer saline (TBS) solution of the antibody (pH, 8.2; concentration, 0.5 to 10 µg/ml) or a 0.05 M carbonate-bicarbonate buffer solution of the antibody (pH, 9.6; concentration, 0.5 to 10 µg/ml) is dripped onto the substrate and allowed to stand for two hours at 20 to 30° C or overnight at 4° C, after which an antibody liquid is sucked by a spoid and removed and subsequently the remnant mass is washed thrice with 100 µl of TBS for physically adsorbing the antibody on the substrate 100. If a substrate having functional groups, such as sulfone groups, amino groups, carboxyl groups or derivatives thereof on its surface is used, the antibody may be chemically bound to the substrate ( see "Methods of biochemical Experiments 11 Enzyme Immunoassay" by P. Tijssen, translated by Eiji Ishikawa, published by Tokyo Kagaku Dojin, and "Monoclonal Antibody, Hybridoma and ELISA", by Tatsuo Iwasaki et al, published by Kodansha).

The substrate for analyses of the present invention is prepared by treating the surface of the substrate having portions carrying thereon the reagents for analysis with an aqueous solution consisting essentially of protein (104).

The aqueous solution consisting essentially of protein may be enumerated by an aqueous solution of bovine serum albumin (BSA), an aqueous solution consisting essentially of casein, an aqueous solution consisting essentially of egg albumin (OVA) and an aqueous solution consisting essentially of fetal calf serum (FCS). These aqueous solutions may also contain such components as sodium azide or glycine.

For treating the substrate with an aqueous solution consisting essentially of protein, the surface 104 of the substrate for analysis, on which the reagent for analysis is fixed, is filled with aqueous solutions of BSA of various concentrations, and allowed to stand at 30° C for one hour, after which the BSA solution is removed and the remnant mass is washed thrice with 100 µl of TBS added with 0.05% Tween-20 (TBS Tween). Such a substrate which has a contact angle in water of 70° or less and preferably 60 to 50° after air-drying is used as a substrate for analysis.

By treating the surface of the substrate for analysis, on which the reagent for analysis is fixed, with the aqueous solution containing the protein, the sample for analysis may be developed smoothly and uniformly without causing reaction fluctuations at the portions of the substrate surface. On the other hand, portions on the disk which is not covered with protein yet may be blocked by the protein-containing aqueous solution to inhibit nonspecific adsorption of the antigen or antibody on the disk surface.

Besides, since the flow rate of the sample for analysis during disk rotation may be adjusted so as to be proportionate to substantially the square of the number of revolutions of the disk, it becomes possible to control the flowing behavior.

The method of analysis of the liquid samples of the present invention is hereinafter explained.

In the method of analysis of the liquid sample according to the present invention, the above-mentioned substrate for analysis of liquid samples according to the present invention is employed.

In the method of the present invention, the liquid sample is dripped and developed on the substrate for analysis (disk-shaped sensor) on which the reagent for analysis is fixed and which has been treated in the above-mentioned protein-containing aqueous solution so that the contact angle with respect to water is 70° , or alternatively, the liquid sample is developed on the substrate by substrate rotation, for reacting the reagent for analysis with the liquid sample.

Although a variety of liquid samples may be analyzed by the present invention, the present invention is most effective for analysis of liquid samples, such as whole blood, blood serum, urine etc. If the whole blood is used for analysis, a membrane filter, not shown, may be provided on the disk 100 and, using the membrane filter, blood corpuscles may be separated from the blood serum, which is employed. Alternatively, the blood serum, freed of blood corpuscles in advance using a centrifugal separator, may also be employed.

There is no particular limitation to the objects of analysis in the liquid samples. These objects of analysis may be enumerated for example by antigens such as C-reactive protein (CRP), α-fetoprotein (AFT) and carcinoembryonic antigen (CEA) and antibodies such as anti-CRP, anti-AFP and anti-CEA.

Meanwhile, according to the present invention, a second reagent for analysis (secondary reagent) may be used after the end of the reaction of the reagent for analysis with the liquid sample to facilitate measurement of the properties of the reaction products.

For example, if the object of analysis is an antigen (antibody), a solution containing antibody (antigen) fixed insoluble carrier particles is used as the second reagent for analysis. The solution containing antibody (antigen) fixed insoluble carrier particles means an antibody (antigen) with respect to the antigen (antibody) as the object of analysis which antibody (antigen) is physically or chemically adsorbed or otherwise fixed to insoluble carrier particles (latex particles), such as plastics particles or colloidal particles etc. These antibody (antigen) fixed insoluble carrier particles are captured by the antigen (antibody) as the object of analysis in a one-for-one relationship. Therefore, if the insoluble carrier particles having a particle size larger than that of the antigen (antibody) as an object of analysis are used, measurement becomes easier than when the number of the antigen (antibody) is counted directly.

Besides, by using the insoluble carrier particles exhibiting fluorescent or dyeing properties, it becomes possible to carry out analysis by taking advantage of these fluorescent or dyeing properties.

According to the present invention, analysis is performed by measuring changes produced by the reaction between the reagent for analysis and the liquid sample on the substrate.

Although there is no limitation to means for measuring the changes produced by the reaction between the reagent for analysis and the liquid samples, optical measurement means are most preferred.

The optical measurement means may be enumerated by those employing such properties as changes in reflectance, absorption of the light of a particular wavelength, intensity of the fluorescent light or rotation of the polarization plane of the polarized light (optical activity).

For directly measuring the number of the object of analysis, such as the antigen or the insoluble carrier particles, a light source such a laser light source or a source of white light may be used as optical measurement means. As a laser light source to be employed, a semiconductor laser, which is used generally, may be directly used, and a suitable laser light source may be selected as a function of the size of the insoluble carrier particles.

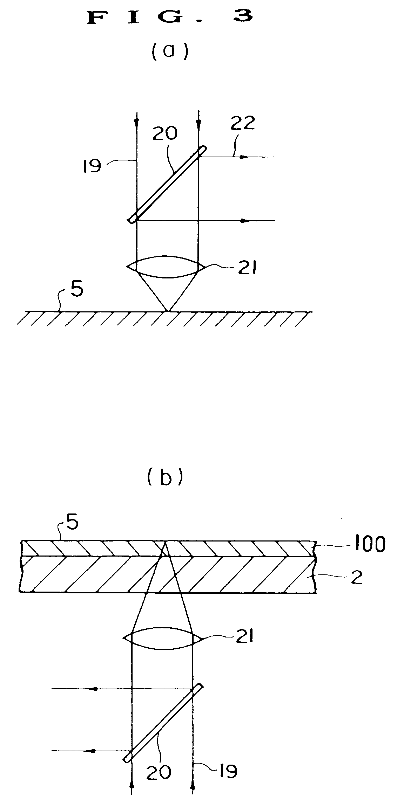

As an analytic device equipped with optical measurement means, a device shown for example in Fig.2 is employed (see JP Patent Application No.1-092367 (1989).

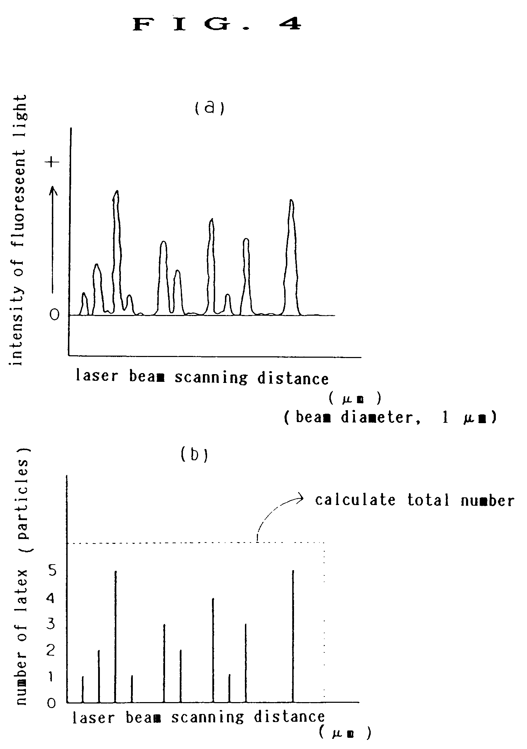

Referring to Fig.2, 100 is a substrate or disk set on a turntable 2. A saucer 6 for receiving a sample dropped from the disk 100 for recovery into a recovery tank 7 is placed at a lower outer periphery of the disk 100. 8 is an electric motor for rotating the disk and 9 is a driving controlling circuit for motor 8. 11 is a nozzle for dripping the sample supplied from a sample supplying device 12 onto the substrate 100. 14 is an optical measurement head which may be reciprocated by a feed screw 15 provided extending along the radius of the disk. 16 is an electric motor for driving the head 14 and 17 a driving controlling circuit for the driving motor 16. The optical measurement head 14 has a laser light projecting part and a laser light receiving part. As shown in Fig.3a, a light 19 projected from the projecting part is radiated via a half mirror 20 and a lens 21 on a sample set on a sample developing surface 5 and a light 22 reflected from the sample is received by the light receiving section. Meanwhile, the light 19 may be projected from the rear side of the substrate, as shown in Fig.3b, while analysis may be performed using a transmitted light instead of the reflected light. In these cases ,the turntable 2 may be formed of a transparent material, or the turntable 2 may be removed. 23 is a signal processing device having the functions of supplying a signal to the head 14 for illuminating the light source and of processing and analyzing light signals received by the head 14. 24 is a display device for displaying the results of analysis on a display screen, and 25 a recorder for printing and outputting the results of analysis. 27 is a central processing unit (CPU) for controlling the sample supplying device 12, driving controlling devices 9 and 17 and the signal processing device 23 for causing the operation as prescribed by the program. 28 is an operating device for the program of CPU 27 and 29 a program memory.

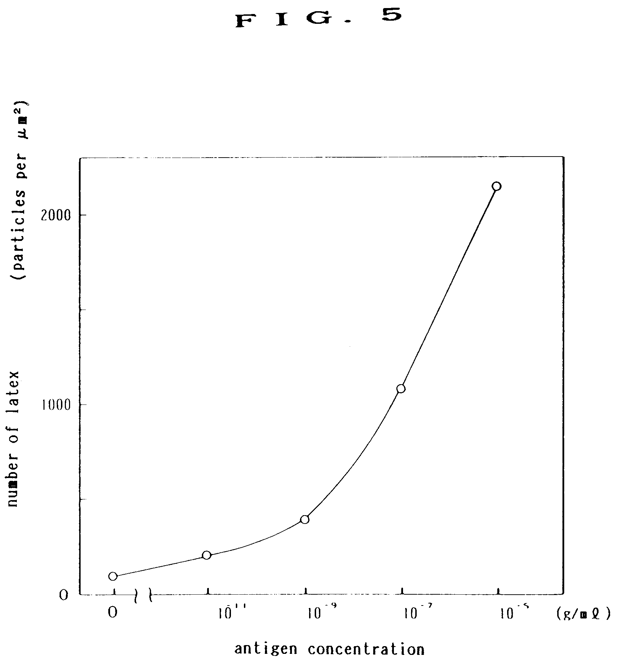

With the above-described device for analysis, the process steps of dispensing the liquid sample of analysis on the disk, developing the sample of analysis, washing of the disk and measurement or analysis, may be carried out in their entirety on the disk, while the analysis may be automated. If the insoluble carrier particles emit fluorescent light, a photodetector is used as the optical measurement means. By the interposition of a filter, the intensities of the fluorescent light at particular wavelengths of e.g. 397 nm, 472 nm, 540 nm or 577 nm, may be detected, as shown in Fig.4a, and the detected intensities may be calculated in terms of the numbers of particles (Fig.4b). For finding the antigen concentration from the measured numbers of the insoluble carrier particles, the procedure described above is followed except using the sample of known antigen concentration to find the relation between the antigen concentration and the numbers of the insoluble carrier particles to prepare a calibration curve in advance to find the antigen concentration based on this calibration curve.

### DESCRIPTION OF THE PREFERRED EMBODIMENT

The present invention will be explained with reference to an Example and a Comparative Example. It is noted that these Examples are given only for the sake of illustration and are not intended for limiting the scope of the invention.

### Example 1

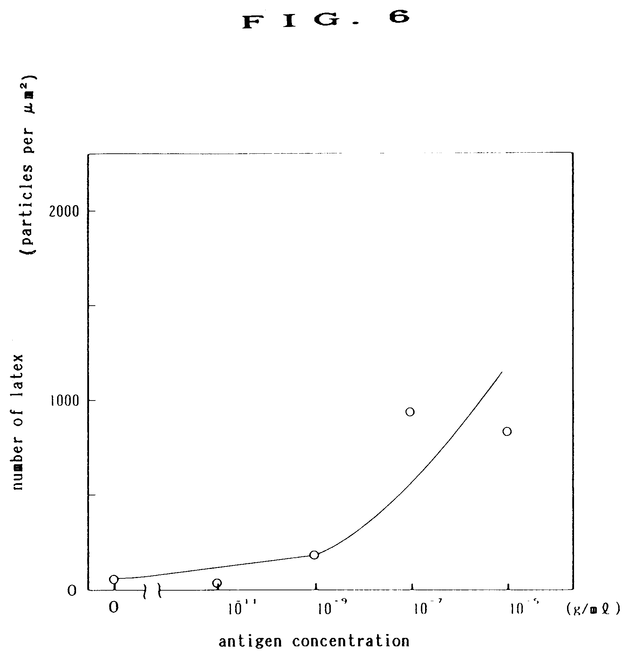

(i) Tris buffer saline (TBS) solution of a CRP antibody produced by a rabbit (concentration, 1 x 10⁻⁵ g/ml) was dripped in amounts of 50 µm at 4 cm radius points of sector-shaped channels of a rotatable polycarbonate substrate shown in Fig.1. After being allowed to stand at room temperature for two hours, the substrate was washed thrice with 100 µl of the TBS solution (0.05 M, pH 8.2) to prepare an antibody-fixed substrate. The height of each rib 100a defining the channels was set to 0.2 mm.
(ii) The flow paths of the respective channels of the antibody-fixed substrate, prepared as above, inclusive of the antibody-fixed portions, were filled with 200 µl of the TBS solution of BSA (V), an aqueous solution of protein for surface processing (BSA concentration, 1%), and the substrate was allowed to stand at 30° C for an hours. After the BSA solution was removed, the remnant mass was washed thrice with 100 µl of the TBS-Tween solution. The resulting substrate, having the contact angle of 52° with respect to water after air dried, was used as a disk for CRP examination (disk-shaped sensor).
(iii) The disk for CRP examination, prepared as above, was set on an analysis device shown in Fig.2. Meanwhile, as an optical head for the analytic device, such a head in which, by the combination of a white light source and a wavelength filter, the light of the wavelength of 485 nm could be selectively taken out and the fluorescent light from the sample having the wavelength of 540 nm could be detected, was employed.
(iv) Then, for preparing a calibration curve, standard serum samples with known CRP concentrations, prepared in TBS (concentrations: 0 g/ml, 1.0x10⁻¹¹ g/ml, 1.0x10⁻⁹ g/ml, 1.0x10⁻⁷ g/ml and 1.0x10⁻⁵ g/ml) were dripped by amounts of 50 µl, in the channels of the disk for CRP examination, prepared as above, and the disk was immediately set into rotation at 100 rpm for one minute. At this time, the serum samples were developed smoothly and uniformly as shown in Fig.7a without producing reaction fluctuations. The rotational velocity of the disk was raised to 500 rpm and kept to be rotated for five seconds for removing the standard serum samples.
(v) 100 µl of the TBS-Tween solution was dripped onto each channel of the disk which was rotated at 100 rpm for one minute. The rotational velocity of the disk was then raised to 500 rpm and kept to be rotated for five second for removing the TBS-Tween. This process was repeated three times.
(vi) A latex solution, having CRP antibodies fixed therein, was dripped in amounts of 50 µl on the channels of the disk and the disk was immediately set into rotation at 100 rpm for one minute. The rotational velocity of the disk was then raised to 500 rpm and the disk was kept to be rotated for five seconds for removing the latex solution.
(vii) The processing similar to that of the process (v) was carried out.
(viii) 200 µl of water was dripped in the respective channels on the disk and the disk was kept to be rotated at 100 rpm for one minute. The rotational velocity of the disk was then raised to 500 rpm and the disk was kept to be rotated for five seconds for removing water. This process was repeated three times.
(ix) The antibody-fixed portions of the disk were scanned by the optical head built into an analysis device and the intensities of the fluorescent light were measured by a photodetector of the optical head. The integrated values of the fluorescent light intensities were calculated in terms of the numbers of latex particles to find the relation between the CRP concentration and the numbers of the latex particles. The results are shown in Table 1 and in Fig.5 which shows a calibration curve.

### Comparative Example

Analysis was performed in the same way as in Example above except that the process (ii) of surface treatment with an aqueous protein solution was not carried out.

It was found that, in the processing operation of the process (iv), that is the process of development of a serum sample, the serum sample was not fluidized when the disk was rotated at a 100 rpm. When the rotational velocity of the disk was raised to 300 rpm, the serum sample was fluidized. However, the locus of movement of the sample was not constant but reaction fluctuations were produced.

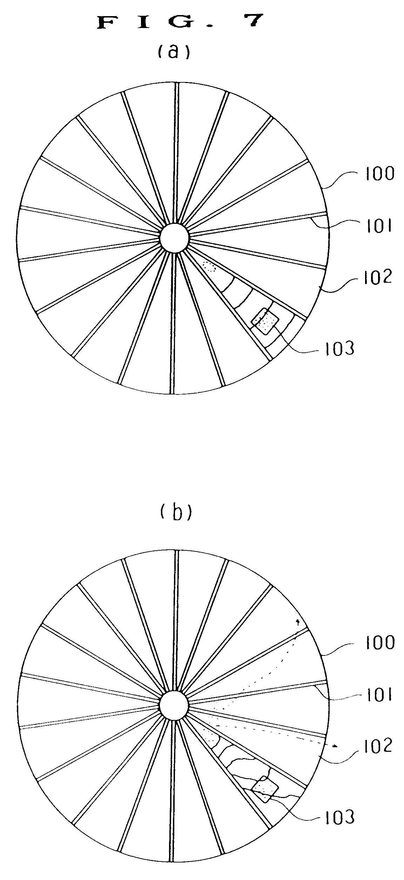

The calibration curve was then found. It was shown that, as may be seen from Fig.6, the calibration curve was not accurate because of excessive fluctuations.

It may be seen from the results of the Example and the Comparative Example that the method for analysis and the substrate for analysis of the liquid samples according to the present invention may give rise to improved fluidity of the sample of analysis on the disk-shaped sensor to improve analysis accuracy and reproducibility.

## Claims

1. A method for analyzing a liquid sample comprising fixing a reagent for analysis on a rotatable substrate, developing a liquid sample on the substrate by dripping or rotation of the substrate for reacting the reagent for analysis with the liquid sample and measuring changes produced by the reaction on said substrate, said method further comprising treating a substrate surface inclusive of portions having said reagent for analysis fixed thereon with an aqueous solution consisting essentially of protein so that the contact angle of substrate with water is not more than 70° , and subsequently analyzing said liquid sample.

2. A method for analyzing a liquid sample as claimed in claim 1 wherein the substrate surface inclusive of portions having said reagent for analysis fixed thereon is treated with the aqueous solution consisting essentially of protein so that the angle of contact of the substrate is in the range of from 60° to 50° .

3. A method for analyzing a liquid sample as claimed in claims 1 or 2 wherein the aqueous solution consisting essentially of protein is an aqueous solution consisting essentially of albumin or casein.

4. A substrate for analysis of a liquid sample characterized in that it is produced by fixing a reagent for analysis on a rotatable disk and by treating a substrate surface inclusive of portions having said reagent for analysis fixed thereon with an aqueous solution consisting essentially of protein so that the contact angle with water is not more than 70°.
